# EUROPEAN PATENT APPLICATION

(11) **EP 4 198 631 A1**
(43) Date of publication of application: **21.06.2023**
(21) Application number: 21215382.9
(22) Date of filing: 17.12.2021
(51) Int. Cl.: G03F 7/20

(54) **METHOD AND APPARATUS FOR CALCULATING A REPARATION DOSE FOR A DIE OF A SUBSTRATE EXPOSED BY A LITHOGRAPHIC APPARATUS**

(71) Applicant: ASML Netherlands B.V., 5500 AH Veldhoven (NL)
(72) Inventor: LI, Jingshi, 5500 AH Veldhoven (NL); CHEN, Po-Ju, 5500 AH Veldhoven (NL)
(74) Representative: ASML Netherlands B.V.

(57) **Abstract**

Method for calculating a reparation dose for a die of a substrate, comprising: a) obtaining a function of a distribution of an energy dose applied to the die over time based on a measurement of exposure energy, b) determining energy dose in a timeslot with a reparation point in the centre of the timeslot, and proceeding with step c) for the timeslot if the energy dose in the timeslot is less than an energy dose limit, c) calculating a slope of the function in the timeslot to determine at least one measurement point, the at least one measurement point being positioned within the timeslot based on the slope of the function in the timeslot; d) calculating a reparation dose at the measurement point; e) calculating a repair energy based on the reparation dose; f) updating the function over the timeslot based on applying the repair energy at the reparation point.

## Description

### FIELD

The present invention relates to a method and apparatus for calculating at least one reparation dose for at least one die of a substrate exposed by a lithographic apparatus. In particular, the present invention relates to a method for calculating a repair energy for the repair based on the reparation dose.

### BACKGROUND

A lithographic apparatus is a machine constructed to apply a desired pattern onto a substrate. A lithographic apparatus can be used, for example, in the manufacture of integrated circuits (ICs). A lithographic apparatus may, for example, project a pattern at a patterning device (e.g., a mask) onto a layer of radiation-sensitive material (resist) provided on a substrate.

To project a pattern on a substrate a lithographic apparatus may use electromagnetic radiation. The wavelength of this radiation determines the minimum size of features which can be formed on the substrate. A lithographic apparatus, which uses extreme ultraviolet (EUV) radiation, having a wavelength within the range 4-20 nm, for example 6.7 nm or 13.5 nm, may be used to form smaller features on a substrate than a lithographic apparatus which uses, for example, radiation with a wavelength of 193 nm.

An EUV lithographic system comprises a radiation source configured to generate EUV radiation and a lithographic apparatus configured to receive the EUV radiation from the lithographic source and to use the EUV radiation to image a pattern onto a substrate (i.e. a semiconductor wafer) provided with a photosensitive resist. The radiation source is typically of the laser-produced-plasma (LPP) source, wherein a high-power laser converts mass-limited fuel targets into plasma, one at the time. The plasma generates EUV. Accordingly, the EUV radiation is provided in a sequence of pulses, at a rate of, e.g., 50 kHz or 100 kHz.

The lithographic apparatus may be a scanner, for example, in which each target area on the substrate may be irradiated by scanning the pattern to be imaged through a radiation beam in a given direction while simultaneously scanning the substrate parallel or anti parallel to this direction. Generally, it is desirable to minimize the time taken to image the pattern onto the substrate so as to increase the throughput of the lithographic apparatus (i.e., the number of substrates per hour).

The amount of radiation energy delivered to a unit of surface area of the substrate is referred to as the dose.

Sometimes, the radiation source provides insufficient energy to the target area being scanned. Such target area may be known as a die. Such target area will then need to be subjected to a re-exposure in a second pass of the substrate through the scanner, as the first pass did not deliver the required amount of energy. Such second pass is also referred to as a "die-repair" or re-expose pass. It is desirable to accurately control the dose of radiation delivered to the substrate during die repair in order to avoid causing an over dose or under dose problem.

### SUMMARY

According to a first aspect of the invention, there is provided a method for calculating at least one reparation dose for at least one die of a substrate exposed by a lithographic apparatus, the method comprising: a) obtaining a function of a distribution of an energy dose applied to the at least one die over time based on a measurement of exposure energy, b) determining energy dose in a timeslot with a reparation point in the centre of the timeslot, and proceeding with step c) for the timeslot if the energy dose in the timeslot is less than an energy dose limit, or proceeding with step g) if the energy dose in the timeslot is not less than the energy dose limit, c) calculating a slope of the function in the timeslot to determine at least one measurement point, the at least one measurement point being positioned within the timeslot based on the slope of the function in the timeslot; d) calculating a reparation dose at the measurement point, the reparation dose being the difference between the exposure energy dose applied at the at least one measurement point and a first energy dose threshold; e) calculating a repair energy based on the reparation dose; f) updating the function over the timeslot based on applying the repair energy at the reparation point; and g) repeating steps b) to f) for the next reparation point until a loop through the function is completed.

In step c), the slope of the function in the timeslot is used to determine at least one measurement point. The slope advantageously provides the necessary information to choose the measurement point which optimises the reparation dose minimizing or avoiding any possible overdose. In other embodiments, other properties of the function may be used for determining the at least one measurement point. Therefore, step c) may be named as: calculating at least one parameter of the function in the timeslot to determine at least one measurement point, the at least one measurement point being positioned within the timeslot based on the at least one parameter of the function in the timeslot. The parameter may be the mean, the median, the slope or other suitable mathematical parameter or statistical parameter. This embodiment may be applied to any of the embodiments of the aspects of the invention and also to any embodiment described in the figures.

An advantage may be that all, or at least the majority of, dies of the substrate may be repaired without an over dose or under dose problem when the radiation (e.g. EUV) source is running at aggressive energy controller.

The slope of the function includes both direction and steepness of the function.

The timeslot is set around the reparation point.

The energy dose limit may be in a range of -1% to 0% dose error.

The method may further comprise setting the measurement point to be earlier in time than a centre of the timeslot when the slope of the function is overall negative in the timeslot. Centre of the timeslot may be the median point within the timeslot.

Negative slope means that the line falls as move forward in time (i.e. left to right in graph with energy dose being on the y-axis and time being on the x-axis).

There may be a linear relationship between steepness of slope and position of the measurement point in the timeslot. That is, the greater the steepness of the slope of the function, the earlier in time the measurement point is positioned.

The method may further comprise setting the measurement point to be later in time than a centre of the timeslot when the slope of the function is overall positive in the timeslot.

Positive slope means that the line rises as move forward in time (i.e. from left to right in graph with energy dose being on the y-axis and time being on the x-axis).

There may be a linear relationship between steepness of slope and position of the measurement point in the timeslot. That is, the greater the steepness of the slope of the function, the later in time the measurement point is positioned.

The method may further comprise setting the measurement point to be in a centre of the timeslot when the function forms a valley in the timeslot.

The function forms a valley means that the slope of the function is neither overall positive nor overall negative, i.e. the slope of the function is negative and then positive in the timeslot as move forward in time (i.e. left to right in graph with energy dose being on the y-axis and time being on the x-axis).

The slope of the function may be determined in a first half of the timeslot and may be determined in a second half of the timeslot.

The determination on whether the slope of the function is overall negative, positive or forms a valley may be taken based on whether the slope of the function was negative or positive in the first half of the timeslot and the second half of the timeslot. For example, if the slope was negative in the first half of the timeslot and negative in the second half of the timeslot, then the slope would be taken to be overall negative in the timeslot. Minor peaks and troughs within the halves of the timeslot may not be taken into account as it is the overall slope of the function in the respective half that is taken.

The method may further comprise setting a second energy dose threshold to be above 0% dose error or maximum energy dose of a peak of the function in the timeslot, and not updating the function if the energy dose in the timeslot would exceed the second energy dose threshold when the repair energy was applied at the reparation point.

A peak of the function means that the slope of the function is positive and then negative in the timeslot as move from left to right (forward in time) - Energy dose being on the y-axis and time being on the x-axis.

This may avoid adding unwanted extra positive dose to an existing peak.

No repair may be allowed when overdose would be generated within timeslot.

The method may further comprise incrementally increasing energy until the second energy dose threshold is reached, and keeping the previously accumulated energy.

This may avoid requesting energy that is too little for the radiation source to deliver which may cause discrepancy on the request energy and the delivered energy.

The energy may be increased per minimal unit.

The method may further comprise setting the first energy dose threshold to be at least one of 0% dose error, in a range of -1 to 0% dose error, in a range of -5% to 0% dose error, in a range of - 10% to 0% dose error.

The method may further comprise setting the first energy dose threshold dependent on maximum energy dose in the timeslot.

The method may further comprise reducing the first energy dose threshold if the maximum energy dose in the timeslot is greater than a predetermined energy dose.

This may avoid an aggressive repair that may cause a new peak and prevent repair later. If repair was prevented later than an under-dose issue could occur.

The method may further comprise setting the first energy dose threshold to be less than 0% dose error if the maximum energy dose in the timeslot is greater than 0% dose error.

The loop may be a first loop and the method may further comprise carrying out steps b) to g) in a second loop through the function opposite to the direction taken in the first loop.

This may repair remaining valleys which followed a relatively large peak in the direction taken in the first loop.

The method may further comprise setting the first energy dose threshold of the second loop to be higher than the first energy dose threshold of the first loop.

This may allow a more aggressive repair in the second loop.

The method may further comprise setting a second energy dose threshold of the second loop to be higher than the second energy dose threshold of the first loop.

This may allow more overdose in the second loop compared with the first loop. This allows a more aggressive repair in the second loop.

The method may further comprise calculating the repair energy by incrementally increasing energy until the first energy dose threshold is reached.

The method may further comprise applying, by a lithographic apparatus, the repair energy at one or more of the reparation points based on the reparation dose to repair the at least one die of the substrate.

The substrate may be exposed, a determination may be made whether repair is needed and then repair may be carried out at end of the exposure of the substrate. The substrate may stay in the substrate table when the determination is made whether repair is needed, i.e. the substrate does not leave the lithographic apparatus during this process.

According to a second aspect of the invention, there is provided a method for repairing at least one die comprising: performing the method for calculating at least one reparation dose as described above, and applying repair energy in at least one part of the die based on the reparation dose calculated.

According to a third aspect of the invention, there is provided a system configured for calculating at least one reparation dose for at least one die of a substrate exposed by a lithographic apparatus, the system configured to: a) obtain a function of a distribution of an energy dose applied to the at least one die over time based on a measurement of exposure energy, b) determine the energy dose in a timeslot with a reparation point in the centre of the timeslot, and proceed with step c) for the timeslot if the energy dose in the timeslot is less than an energy dose limit, or proceed with step g) if the energy dose in the timeslot is not less than the energy dose limit, c) calculate a slope of the function in the timeslot to determine at least one measurement point, the at least one measurement point being positioned within the timeslot based on the slope of the function in the timeslot; d) calculate a reparation dose at the measurement point, the reparation dose being the difference between the exposure energy dose applied at the at least one measurement point and a first energy dose threshold; e) calculate a repair energy based on the reparation dose; f) update the function over the timeslot based on applying the repair energy at the reparation point; and g) repeat steps b) to f) for the next reparation point until a loop through the function is completed.

According to a fourth aspect of the invention, there is provided a lithographic apparatus comprising a projection system configured to project a EUV or DUV radiation beam to project a pattern from a patterning device onto a substrate, wherein the lithographic apparatus comprises the system described above.

According to a fifth aspect of the invention, there is provided a computer program comprising computer readable instructions configured to cause a processor to carry out a method as described above.

According to a sixth aspect of the invention, there is provided a computer readable medium carrying a computer program as described above.

According to a seventh aspect of the invention, there is provided a computer apparatus comprising: a memory storing processor readable instructions; and a processor arranged to read and execute instructions stored in said memory; wherein said processor readable instructions comprise instructions arranged to control the computer to carry out a method as described above.

### BRIEF DESCRIPTION OF THE DRAWINGS

Embodiments of the invention will now be described, by way of example only, with reference to the accompanying schematic drawings, in which:
- Figure 1 depicts a lithographic system comprising a lithographic apparatus and a radiation source;
- Figure 2 depicts a graph of repair energy (Eᵣₑₚₐᵢᵣ) over time and a graph of energy dose (Dose) over time when that repair energy is applied.
- Figure 3 depicts a graph of energy dose over time;
- Figure 4 depicts a graph of energy dose over time with a measurement point in the centre of a timeslot;
- Figure 5 depicts a graph of energy dose over time with a dynamic measurement point earlier than the centre of a timeslot and with a first energy dose threshold;
- Figure 6 depicts a flow diagram of a method of calculating reparation dose for a die of a substrate exposed by a lithographic apparatus;
- Figure 7 depicts a graph of energy dose over time with a dynamic measurement point set in the centre of a timeslot;
- Figure 8 depicts a graph of energy dose over time with a second energy dose threshold;
- Figure 9 depicts a graph of energy dose over time with a dynamic first energy dose threshold;
- Figure 10 depicts a graph of energy dose over time for a second loop.

### DETAILED DESCRIPTION

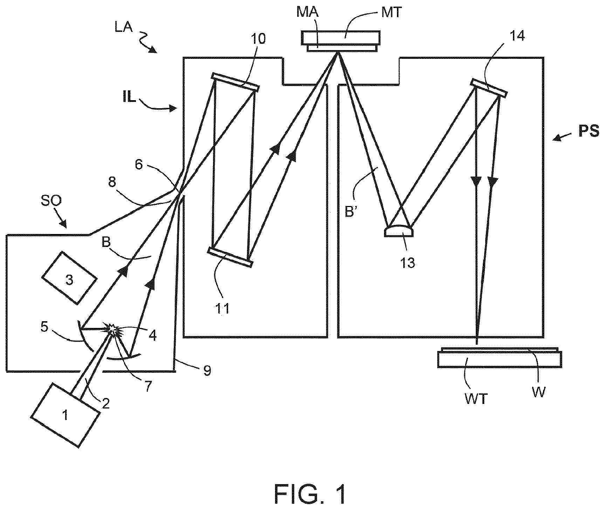

Figure 1 shows a lithographic system comprising a radiation source SO and a lithographic apparatus LA. The radiation source SO is configured to generate an EUV radiation beam B and to supply the EUV radiation beam B to the lithographic apparatus LA. The lithographic apparatus LA comprises an illumination system IL, a support structure MT configured to support a patterning device MA (e.g., a mask), a projection system PS and a substrate table WT configured to support a substrate W.

The illumination system IL is configured to condition the EUV radiation beam B before the EUV radiation beam B is incident upon the patterning device MA. Thereto, the illumination system IL may include a facetted field mirror device 10 and a facetted pupil mirror device 11. The faceted field mirror device 10 and faceted pupil mirror device 11 together provide the EUV radiation beam B with a desired cross-sectional shape and a desired intensity distribution. The illumination system IL may include other mirrors or devices in addition to, or instead of, the faceted field mirror device 10 and faceted pupil mirror device 11.

After being thus conditioned, the EUV radiation beam B interacts with the patterning device MA. As a result of this interaction, a patterned EUV radiation beam B' is generated. The projection system PS is configured to project the patterned EUV radiation beam B' onto the substrate W. For that purpose, the projection system PS may comprise a plurality of mirrors 13,14 which are configured to project the patterned EUV radiation beam B' onto the substrate W held by the substrate table WT. The projection system PS may apply a reduction factor to the patterned EUV radiation beam B', thus forming an image with features that are smaller than corresponding features on the patterning device MA. For example, a reduction factor of 4 or 8 may be applied. Although the projection system PS is illustrated as having only two mirrors 13,14 in Figure 1, the projection system PS may include a different number of mirrors (e.g., six or eight mirrors).

The substrate W may include previously formed patterns. Where this is the case, the lithographic apparatus LA aligns the image, formed by the patterned EUV radiation beam B', with a pattern previously formed on the substrate W.

A relative vacuum, i.e. a small amount of gas (e.g. hydrogen) at a pressure well below atmospheric pressure, may be provided in the radiation source SO, in the illumination system IL, and/or in the projection system PS.

The radiation source SO shown in Figure 1 is, for example, of a type which may be referred to as a laser produced plasma (LPP) source. A laser system 1, which may, for example, include a CO₂ laser, is arranged to deposit energy via a laser beam 2 into a fuel, such as tin (Sn) which is provided from, e.g., a fuel emitter 3. Although tin is referred to in the following description, any suitable fuel may be used. The fuel may, for example, be in liquid form, and may, for example, be a metal or alloy. The fuel emitter 3 may comprise a nozzle configured to direct tin, e.g. in the form of droplets, along a trajectory towards a plasma formation region 4. The laser beam 2 is incident upon the tin at the plasma formation region 4. The deposition of laser energy into the tin creates a tin plasma 7 at the plasma formation region 4. Radiation, including EUV radiation, is emitted from the plasma 7 during de-excitation and recombination of electrons with ions of the plasma.

The EUV radiation from the plasma is collected and focused by a collector 5. Collector 5 comprises, for example, a near-normal incidence radiation collector 5 (sometimes referred to more generally as a normal-incidence radiation collector). The collector 5 may have a multilayer mirror structure which is arranged to reflect EUV radiation (e.g., EUV radiation having a desired wavelength such as 13.5 nm). The collector 5 may have an ellipsoidal configuration, having two focal points. A first one of the focal points may be at the plasma formation region 4, and a second one of the focal points may be at an intermediate focus 6, as discussed below.

The laser system 1 may be spatially separated from the radiation source SO. Where this is the case, the laser beam 2 may be passed from the laser system 1 to the radiation source SO with the aid of a beam delivery system (not shown) comprising, for example, suitable directing mirrors and/or a beam expander, and/or other optics. The laser system 1, the radiation source SO and the beam delivery system may together be considered to be a radiation system.

Radiation that is reflected by the collector 5 forms the EUV radiation beam B. The EUV radiation beam B is focused at intermediate focus 6 to form an image at the intermediate focus 6 of the plasma present at the plasma formation region 4. The image at the intermediate focus 6 acts as a virtual radiation source for the illumination system IL. The radiation source SO is arranged such that the intermediate focus 6 is located at or near to an opening 8 in an enclosing structure 9 of the radiation source SO.

Although Figure 1 depicts the radiation source SO as a laser produced plasma (LPP) source, any suitable source such as a discharge produced plasma (DPP) source or a free electron laser (FEL) may be used to generate EUV radiation.

The radiation source SO of the lithographic apparatus is operated with a so-called dose margin that controllably sets the level of energy of the radiation (e.g. EUV) generated, averaged over a particular length of time (also referred to as: controlled energy or power), below the source's maximum output power (also referred to as: open-loop energy). A non-zero dose margin enables to compensate for temporally short drops in EUV energy, e.g., by adjusting the duration of one or more pulses, or by adjusting the energy of one or more laser pulses fired on one or more of the next fuel droplets.

In case too many consecutive EUV pulses deliver too low an energy to the substrate's exposed target area, the eventual dose delivered to the target area may be too low.

In practice, the output of a pulsed radiation source SO will vary with time. If the energy of an individual pulse is higher or lower than a nominal or desired output energy for each pulse then this will contribute to a dose error. The fewer pulses received by a target area, the greater the effect of such an individual pulse energy error will be on the total dose.

If the dose margin is made smaller and, therefore, if the radiation source SO is operating closer to maximum power, then the time to expose a single substrate W can be decreased if the scan speed is increased. However, operating the source SO with closer to maximum power also increases the chance of having to re-expose certain target areas of the substrate W in a second pass. The reason for this is that there is not always enough energy available in the limited number of subsequent EUV pulses incident on the target area in order to compensate for a drop in EUV energy delivered by preceding EUV pulses to that target area.

Using more radiation source power, i.e. lower dose margin to yield more dies in a particular time, may be referred to as an source aggressive energy controller method. Aggressive energy controller method in this case allows the source to compensate when a temporal EUV generation drop out happens. The method to boost more power per pulse. This could be done by hitting more droplets when it experienced a EUV generation 'drop out' or 'under dose'. This would reduce the chance of having an under dose error such that the yield of die would be improved. However, when the 'drop out' is too big to be compensated with following pulses (within a slit time slot), it would appear as a 'under dose error' followed with a 'positive bump (or peak)'. This makes it more challenging to calculate a repair.

Previously, the die repair algorithm was a simple integration method that accumulates the missing energy to decide if a repair of energy is needed to fix a dose error of the die. However, this die repair algorithm does not work with the source aggressive energy controller method that could cause over dose or under dose problem. This is because it does not consider where the repair should be taken place and how much. When measurements are only taken in the energy domain, a repair need is only decided when there is sufficient missing energy. However that repair could lead to over dose or insufficient repair in the dose domain. Thus, using this die repair algorithm means that failure rate increases when the source is running at aggressive energy controller.

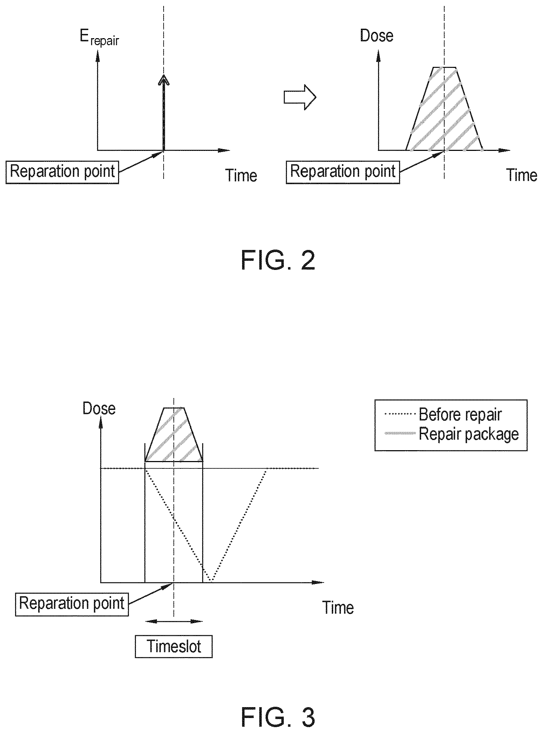

Figure 2 shows a graph of repair energy (Eᵣₑₚₐᵢᵣ) over time and a graph of energy dose (Dose) over time when that repair energy is applied. As can be seen from these graphs, applying repair energy at a particular point in time (which may be referred to as a reparation point) gives a "slit shaped" dose around that reparation point. Thus, the full slit size should be taken into account when an evaluation is made whether a repair to the substrate W should be made or not (i.e. whether there should be a second exposure at this point).

Figure 3 shows a graph of energy dose on the y-axis and time on the x-axis. A repair window or timeslot is set around the reparation point (with the reparation point being in the centre of the timeslot. The original dose error ("before repair") is shown in the graph. The original energy dose applied to the die in the substrate W is based on a measurement of exposure energy. In embodiments, the energy doses within the timeslot are used to evaluate if the repair should be made or not. The "repair package" is shown (i.e. the slit shaped dose), which is centred around the reparation point.

In embodiments, an algorithm is used to loop through the original dose error and calculate the repair for the die of the substrate W. Dose error may be expressed as a percentage with respect to a target or intended dose for the substrate W. For example, 0% dose error may correspond to no difference between the dose and the target dose, whilst -1% dose error may correspond to the dose being -1% below the target dose.

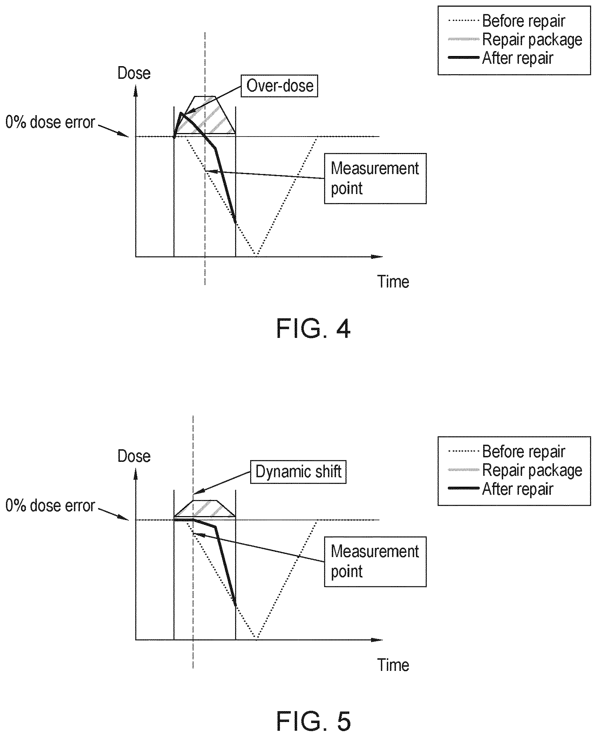

Figure 4 shows a graph of energy dose over time in which a measurement point is taken at the point where the "before repair" dose meets the line in the centre of the timeslot (i.e. which corresponds to the reparation point). The "repair package" is shown (i.e. the slit shaped dose), which is centred around the reparation point. The repair package is based on a reparation dose, the reparation dose being the difference between the exposure energy dose applied at the measurement point and a first energy dose threshold (which may be e.g. 0% dose error).

The repair package would be provided by a particular repair energy being applied at the reparation point (the repair energy having been calculated from the reparation dose). In addition, the "after repair" dose is shown for this repair package, as well as the "before repair" dose. However, in this example, if the repair is requested based on the measurement point being in the centre of the timeslot, it would cause over-dose towards the start of the timeslot as shown. This is because the repair energy is based on the relatively large difference between the exposure energy dose applied at the measurement point and the first energy dose threshold (i.e. 0% dose error in this example), whereas, towards the start of the timeslot, this difference is less. As can be seen in Figure 4, in the centre of the timeslot (i.e. at the measurement/reparation point), the repair dose is at 0% dose error. Thus, in this example, if the repair is requested using the measure in the centre of the timeslot, then there would be over repair to "the past" due to the "slit shaped" dose around the reparation point.

Figure 5 shows a graph of energy dose over time in which a measurement point is taken at a point earlier in time than that shown in Figure 4 (i.e. earlier than the centre of the timeslot). This may be referred to as a dynamic measurement point and there is a dynamic shift as shown. The measurement point in Figure 5 is positioned within the timeslot based on the slope of the before repair dose (i.e. a function of a distribution of an energy dose applied to the at least one die over time) in the timeslot. The slope of the function includes both direction and steepness of the function. That is, whether the function is increasing or decreasing over time and by how much.

In Figure 5, there is a negative slope in the timeslot, i.e. the function is decreasing over time in the timeslot. Negative slope means that the line falls as move forward in time (i.e. left to right in graph). Thus, when the slope of the function is overall negative in the timeslot, then the dynamic measurement point is set to be earlier in time than a centre of the timeslot. There is a linear relationship between steepness of slope and position of the dynamic measurement point in the timeslot. That is, the greater the steepness of the (negative) slope of the function, the earlier in time the dynamic measurement point is positioned.

It can be seen from Figure 5 that, now that the dynamic measurement point is earlier in time than the centre of the timeslot, the reparation dose (i.e. the difference between the exposure energy dose applied at the dynamic measurement point and the first energy dose threshold, i.e. 0% dose error in this example) is less than when taken using the measurement point of Figure 4. Thus, in this example, when the repair is requested using the reparation dose for the dynamic measurement point, then the repair energy is less than when using the measurement point in the centre of the timeslot, and there would not be over repair towards the start of the timeslot. This is illustrated by the magnitude of the "repair package" in dose being less than in Figure 4. As can be seen in Figure 5, the "after repair" dose does not go above the 0% dose error when the function is updated over timeslot based on applying the repair energy at the reparation point.

It will be appreciated that, in embodiments, the first energy dose threshold may be different from the 0% dose error. For example, in embodiments, the first energy dose threshold may be in a range of -1 to 0% dose error, in a range of -5% to 0% dose error, or in a range of -10% to 0% dose error.

When there is more dose error in the future, the measurement is shifted to earlier, i.e. fire less now to avoid over repair. Thus, the dynamic measurement point is used to avoid early fire induced over-dose.

In some embodiments, a dynamic measurement point is taken at a point later in time than the centre of the timeslot. In this case, the slope of the function has been calculated to be overall positive in the timeslot, i.e. the function is increasing over time in the timeslot. Positive slope means that the line rises as move forward in time (i.e. from left to right in graph with energy dose being on the y-axis and time being on the x-axis). Thus, when the slope of the function is overall positive in the timeslot, then the dynamic measurement point is set to be later in time than a centre of the timeslot. There is a linear relationship between steepness of slope and position of the dynamic measurement point in the timeslot. That is, the greater the steepness of the (positive) slope of the function, the later in time the dynamic measurement point is positioned.

Therefore, similarly as in the example of Figure 5 when the dynamic measurement point was moved earlier for a negative slope to avoid over repair near the start of the timeslot, moving the dynamic measurement point to later for a positive slope avoids over repair near the end of the timeslot.

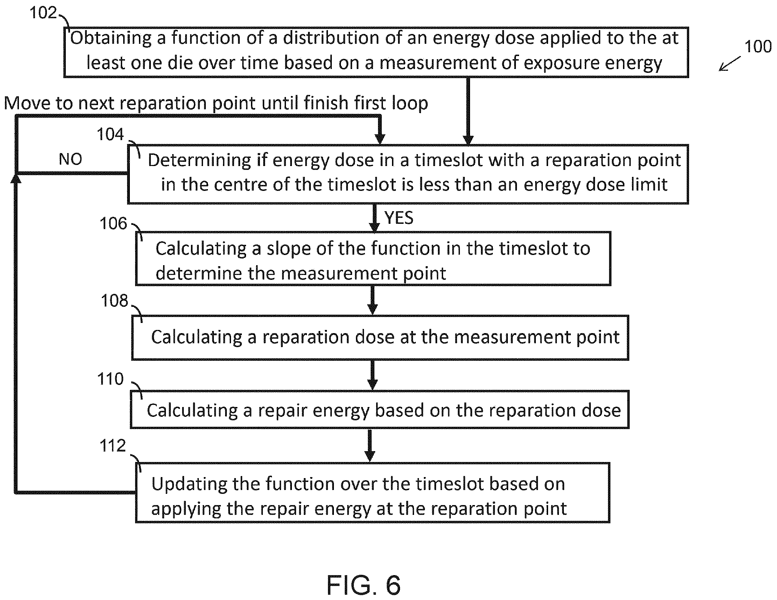

Figure 6 shows a flow-chart describing a method 100 of calculating reparation dose for a die of a substrate W exposed by a lithographic apparatus. An algorithm is used to carry out this method.

Step 102 is obtaining a function of a distribution of an energy dose applied to the at least one die over time based on a measurement of exposure energy.

Step 104 is determining if energy dose in a timeslot with a reparation point in the centre of the timeslot is less than an energy dose limit. If, YES, then move to step 106. If NO, then move to next reparation point and follow Step 104 again. This means that the reparation dose/energy repair calculation for that reparation point is only carried out if any energy dose within the timeslot for that reparation point is less than the energy dose limit. The limit may be considered to be a threshold. In embodiments, the energy dose limit may be in a range of -1% to 0% dose error. For example, the calculation may only be carried out when the energy dose anywhere within the timeslot is <-1% dose error . However, this is just an example, and it will be appreciated that different ranges could be used.

Step 106 is calculating a slope of the function in the timeslot to determine the measurement point. The measurement point being positioned within the timeslot based on the slope of the function in the timeslot.

Step 108 is calculating a reparation dose at the measurement point. The reparation dose is the difference between the exposure energy dose applied at the measurement point and a first energy dose threshold.

Step 110 is calculating a repair energy based on the reparation dose. In embodiments, the repair energy may be calculated by incrementally increasing energy until the first energy dose threshold is reached.

Step 112 is updating the function over the timeslot based on applying the repair energy at the reparation point.

After Step 112, then move to the next reparation point and return to Step 104 until a first loop through the function is completed.

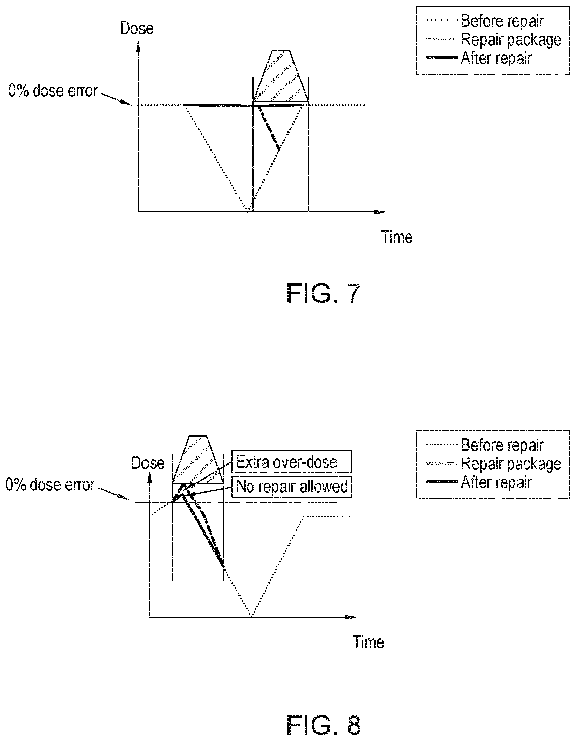

Figure 7 shows a graph of energy dose over time in which the function forms a valley in the timeslot - the dashed line showing the dose after repair for the previous reparation points but before the repair for the reparation point for the timeslot shown. The function forms a valley means that the slope of the function is neither overall positive nor overall negative, i.e. the slope of the function is negative and then positive in the timeslot as move forward in time (i.e. left to right in the graph). In this case, the dynamic measurement point is again positioned within the timeslot based on the slope of the function in the timeslot. The dynamic measurement point is set to be in the centre of the timeslot when the function forms a valley in the timeslot, i.e. fire more now as there is less risk of over repair.

In embodiments, the slope of the function is determined in a first half of the timeslot and is determined in a second half of the timeslot. The determination on whether the slope of the function is overall negative, positive or forms a valley may be taken based on whether the slope of the function was negative or positive in the first half of the timeslot and the second half of the timeslot. For example, if the slope was negative in the first half of the timeslot and negative in the second half of the timeslot, then the slope would be taken to be overall negative in the timeslot. As another example, in the situation when the slope was negative in the first half of the timeslot and positive in the second half of the timeslot, then the function would be taken to form a valley in the timeslot. Minor peaks and troughs within the halves of the timeslot may not be taken into account as it is the overall slope of the function in the respective half that is taken. In embodiments, the sum of the first half of the timeslot and the sum of the second half of the timeslot may be compared and divided with a fixed range. This may be a relatively easier/faster calculation which is important as it is a time critical situation.

Figure 8 shows a graph of energy dose over time in which the "before repair" dose was already above the 0% dose error and there is a peak in the function. In embodiments, the method includes setting a second energy dose threshold be above 0% dose error or maximum energy dose of a peak of the function in the timeslot. As an example, the second energy dose threshold may be set to 5% dose error, or between 5% and 0% or less than 1%, or less than 0.5%. The second energy dose threshold may be tuned to optimize performance. If the energy dose in the timeslot (i.e. anywhere within the timeslot) would exceed the second energy dose threshold when the repair energy was applied at the reparation point, then the function is not updated. In other words, no repair is allowed when overdose would be generated within the timeslot (i.e. limiting the repair energy makes sure that no after-repair dose would exceed the second energy dose threshold). This avoids adding unwanted extra positive dose to an existing peak.

To repair a big dip in the dose, a large repair energy could be asked, which could add an extra positive dose to existing peaks. Even if the peak is still within specification, it is risky because source energy control repro could make the peak into an over-dose error. Thus, the clipping of the repair power is introduced, such that it would never exceed a defined limit or existing peaks.

As can be seen in Figure 8, if the repair was allowed, the "after repair" dose would follow the dashed line - i.e. a larger peak would be produced and thus there would be more dose over the 0% dose error. Instead, no repair is allowed above the second energy dose threshold and the "after repair" dose is the same as the "before repair" dose in this example.

In embodiments, the repair energy is gradually increased until the second energy dose threshold is reached. Energy may be incrementally increased per minimal unit (e.g. an EUV pulse energy) until the second energy dose threshold is reached but the previous accumulated energy would be kept. Thus, some repair could be carried out just as long as it didn't go above the second energy dose threshold.

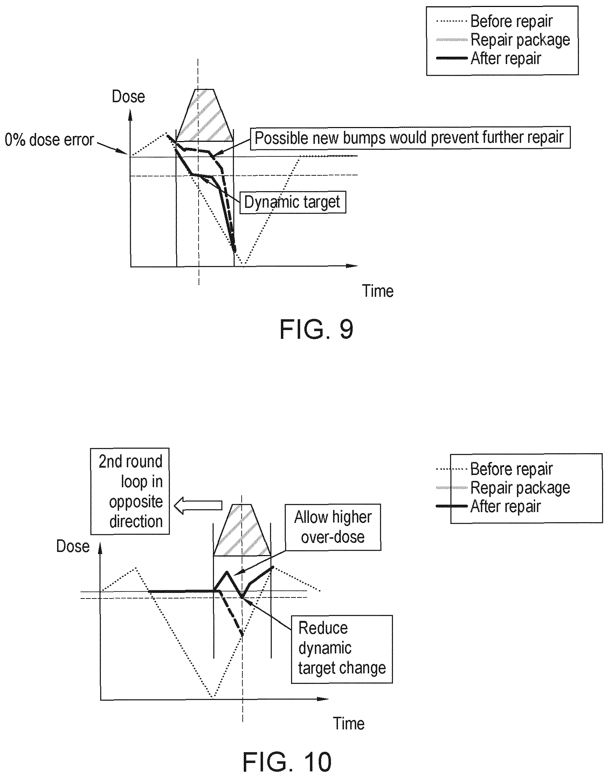

Figure 9 shows a graph of energy dose over time in which the first energy dose threshold (or dynamic target) is changed (i.e. reduced) below the 0% dose error if the maximum energy dose in the timeslot would be greater than 0% dose error when the repair energy is applied at the reparation point. The maximum energy dose in the timeslot may be used with a scaling factor to determine the new target. This may be referred to as a dynamic first energy dose threshold and there is a dynamic shift from 0% dose error as shown. As can be seen from Figure 9, if the repair was carried out using 0% dose error as the first energy dose threshold, then the "after repair" dose would follow the dashed line - i.e. a new peak (or bump) may be produced that could prevent repair later. Instead, the first energy dose threshold is reduced below the 0% dose error and the "after repair" dose is thus lower than it would be if the first energy dose threshold was maintained at 0% dose error. This is because the reparation dose will be reduced. If repair was prevented later then an under-dose issue could occur (e.g. if the new peak is close to the second energy dose threshold). A dynamic repair target is used to lower the repair target (i.e. first energy dose threshold) when there is a relatively big peak in the timeslot, so that less new peak is introduced.

More generally, the first energy dose threshold may be reduced if the maximum energy dose in the timeslot is greater than a predetermined energy dose when the repair energy was applied at the reparation point. More generally still, the first energy dose threshold may be set dependent on the maximum energy dose in the timeslot.

Once the first loop through the function is completed (i.e. the calculations have been carried out by the algorithm for each reparation point), then steps 104-112 are carried out in a second loop through the function opposite to the direction taken in the first loop through the function.

Figure 10 shows a graph of energy dose over time showing the second loop through the function in opposite direction taken in the first loop through the function. There could be remaining valleys after the first loop that require a more aggressive repair. Since the second loop starts from the end of the first loop and continues in the opposite direction to the first loop, then remaining valleys, which followed a relatively large peak in the direction taken in the first loop, may be repaired.

In embodiments, a first energy dose threshold of the second loop is set to be higher than the first energy dose threshold of the first loop. For example, there may be a reduction in the dynamic target change (e.g. that was used as in Figure 9). This allows a more aggressive repair in the second loop when compared to the first loop.

In addition, in embodiments, a second energy dose threshold of the second loop is set to be higher than the second energy dose threshold of the first loop. For example, there may be an increase in the second energy dose threshold (e.g. that was used as in Figure 8). For example, the second energy dose threshold may be set to be higher above 0% dose error than was set in the first loop. In other examples, the second energy dose threshold may be higher than 0.5% dose error or higher than 1% dose error. This allows more overdose in the second loop compared with the first loop and allows a more aggressive repair in the second loop when compared to the first loop. The number of reparation points in the second loop is much less when compared to the first loop. Most of the time, the second loop is not required. Thus the chance of impacting the next reparation point is low. Since it rarely happens, the risk of causing overdose with source control is limited. Furthermore, the limit (second energy dose threshold) may be still smaller than typical dose error in original dose profile.

Once the algorithm has completed the first loop through the function and, in embodiments, completed the second loop through the function, the total repair energy for each reparation point has been calculated based on the reparation dose. The repair energy for the reparation point calculated in the first loop is added to the repair energy for that reparation point calculated in the second loop (if required) to get the total repair energy for that reparation point. This (total) repair energy for each reparation point may then be used in the lithographic apparatus LA to repair the die of the substrate W. That is, in embodiments, the method may include applying, by the lithographic apparatus, the repair energy at the reparation points based on the reparation dose to repair the die of the substrate W.

In embodiments, the substrate W is exposed, a determination is made whether repair is needed (using the algorithm of the present invention) and then repair is carried out at end of the exposure of the substrate W. The substrate W stays in the substrate table WT when the determination is made whether repair is needed, i.e. the substrate W does not leave the lithographic apparatus LA during this process.

It can be shown in simulations and in practice that the new method may repair 100% with the aggressive source controller method (i.e. it's 100% within the specified dose range) while, previously, methods could only repair <40% for most aggressive case. The die repair algorithm of the present invention may have an advantage that all, or at least the majority of, dies of the substrate W may be repaired without an over dose or under dose problem when the radiation (e.g. EUV) source is running at aggressive energy controller.

Although specific reference may be made in this text to the use of lithographic apparatus in the manufacture of ICs, it should be understood that the lithographic apparatus described herein may have other applications. Possible other applications include the manufacture of integrated optical systems, guidance and detection patterns for magnetic domain memories, flat-panel displays, liquid-crystal displays (LCDs), thin-film magnetic heads, etc.

Although specific reference may be made in this text to embodiments of the invention in the context of a lithographic apparatus, embodiments of the invention may be used in other apparatus. Embodiments of the invention may form part of a mask inspection apparatus, a metrology apparatus, or any apparatus that measures or processes an object such as a wafer (or other substrate) or mask (or other patterning device). These apparatus may be generally referred to as lithographic tools. Such a lithographic tool may use vacuum conditions or ambient (non-vacuum) conditions.

Although specific reference may have been made above to the use of embodiments of the invention in the context of optical lithography, it will be appreciated that the invention, where the context allows, is not limited to optical lithography and may be used in other applications, for example imprint lithography.

Where the context allows, embodiments of the invention may be implemented in hardware, firmware, software, or any combination thereof. Embodiments of the invention may also be implemented as instructions stored on a machine-readable medium, which may be read and executed by one or more processors. A machine-readable medium may include any mechanism for storing or transmitting information in a form readable by a machine (e.g., a computing device). For example, a machine-readable medium may include read only memory (ROM); random access memory (RAM); magnetic storage media; optical storage media; flash memory devices; electrical, optical, acoustical or other forms of propagated signals (e.g. carrier waves, infrared signals, digital signals, etc.), and others. Further, firmware, software, routines, instructions may be described herein as performing certain actions. However, it should be appreciated that such descriptions are merely for convenience and that such actions in fact result from computing devices, processors, controllers, or other devices executing the firmware, software, routines, instructions, etc. and in doing that may cause actuators or other devices to interact with the physical world.

While specific embodiments of the invention have been described above, it will be appreciated that the invention may be practiced otherwise than as described. The descriptions above are intended to be illustrative, not limiting. Thus it will be apparent to one skilled in the art that modifications may be made to the invention as described without departing from the scope of the clauses set out below.
1. A method for calculating at least one reparation dose for at least one die of a substrate exposed by a lithographic apparatus, the method comprising:
   a) obtaining a function of a distribution of an energy dose applied to the at least one die over time based on a measurement of exposure energy,
   b) determining energy dose in a timeslot with a reparation point in the centre of the timeslot, and proceeding with step c) for the timeslot if the energy dose in the timeslot is less than an energy dose limit, or proceeding with step g) if the energy dose in the timeslot is not less than the energy dose limit,
   c) calculating at least one parameter of the function in the timeslot to determine at least one measurement point, the at least one measurement point being positioned within the timeslot based on the at least one parameter of the function in the timeslot, more preferably, the at least one parameter of the function is the slope of the function in the timeslot;
   d) calculating a reparation dose at the measurement point, the reparation dose being the difference between the exposure energy dose applied at the at least one measurement point and a first energy dose threshold;
   e) calculating a repair energy based on the reparation dose;
   f) updating the function over the timeslot based on applying the repair energy at the reparation point; and
   g) repeating steps b) to f) for the next reparation point until a loop through the function is completed.
2. The method of clause 1, further comprising setting the measurement point to be earlier in time than a centre of the timeslot when the slope of the function is overall negative in the timeslot.
3.The method of any preceding clause, further comprising setting the measurement point to be later in time than a centre of the timeslot when the slope of the function is overall positive in the timeslot.
4. The method of any preceding clause, further comprising setting the measurement point to be in a centre of the timeslot when the function forms a valley in the timeslot.
5. The method of any preceding clause, wherein the slope of the function is determined in a first half of the timeslot and is determined in a second half of the timeslot.
6.The method of any preceding clause, further comprising setting a second energy dose threshold to be above 0% dose error or maximum energy dose of a peak of the function in the timeslot, and not updating the function if the energy dose in the timeslot would exceed the second energy dose threshold when the repair energy was applied at the reparation point.
7.The method of clause 6, further comprising incrementally increasing energy until the second energy dose threshold is reached, and keeping the previously accumulated energy.
8.The method of any preceding clause, further comprising setting the first energy dose threshold to be at least one of 0% dose error, in a range of -1 to 0% dose error, in a range of -5% to 0% dose error, in a range of -10% to 0% dose error.
9.The method of any of clauses 1-7, further comprising setting the first energy dose threshold dependent on maximum energy dose in the timeslot.
10.The method of clause 9, further comprising reducing the first energy dose threshold if the maximum energy dose in the timeslot is greater than a predetermined energy dose.
11.The method of either of clauses 9 or 10, further comprising setting the first energy dose threshold to be less than 0% dose error if the maximum energy dose in the timeslot is greater than 0% dose error.
12.The method of any preceding clause, wherein the loop is a first loop and the method further comprising carrying out steps b) to g) in a second loop through the function opposite to the direction taken in the first loop.
13.The method of clause 12, further comprising setting the first energy dose threshold of the second loop to be higher than the first energy dose threshold of the first loop.
14.The method of either of clauses 11 or 12, further comprising setting a second energy dose threshold of the second loop to be higher than the second energy dose threshold of the first loop.
15.The method of any preceding clause, further comprising calculating the repair energy by incrementally increasing energy until the first energy dose threshold is reached.
16.The method of any preceding clause, further comprising applying, by a lithographic apparatus, the repair energy at one or more of the reparation points based on the reparation dose to repair the at least one die of the substrate.
17.A method for repairing at least one die comprising:
   performing the method for calculating at least one reparation dose of any preceding clause, and
   applying repair energy in at least one part of the die based on the reparation dose calculated
18.A system configured for calculating at least one reparation dose for at least one die of a substrate exposed by a lithographic apparatus, the system configured to:
   a) obtain a function of a distribution of an energy dose applied to the at least one die over time based on a measurement of exposure energy,
   b) determine the energy dose in a timeslot with a reparation point in the centre of the timeslot, and proceed with step c) for the timeslot if the energy dose in the timeslot is less than an energy dose limit, or proceed with step g) if the energy dose in the timeslot is not less than the energy dose limit,
   c) calculate a least one parameter of the function in the timeslot to determine at least one measurement point, the at least one measurement point being positioned within the timeslot based on the at least one parameter of the function in the timeslot, more preferably, the at least one parameter of the function is the slope of the function in the timeslot;
   d) calculate a reparation dose at the measurement point, the reparation dose being the difference between the exposure energy dose applied at the at least one measurement point and a first energy dose threshold;
   e) calculate a repair energy based on the reparation dose;
   f) update the function over the timeslot based on applying the repair energy at the reparation point; and
   g) repeat steps b) to f) for the next reparation point until a loop through the function is completed.
19.A lithographic apparatus comprising a projection system configured to project a EUV or DUV radiation beam to project a pattern from a patterning device onto a substrate, wherein the lithographic apparatus comprises the system of clause 18.
20.A computer program comprising computer readable instructions configured to cause a processor to carry out a method according to any one of clause 1 to 17.
21.A computer readable medium carrying a computer program according to clause 20.
22.A computer apparatus comprising:
   a memory storing processor readable instructions; and
   a processor arranged to read and execute instructions stored in said memory;
   wherein said processor readable instructions comprise instructions arranged to control the computer to carry out a method according to any one of clauses 1 to 17.

## Claims

1. A method for calculating at least one reparation dose for at least one die of a substrate exposed by a lithographic apparatus, the method comprising:
a) obtaining a function of a distribution of an energy dose applied to the at least one die over time based on a measurement of exposure energy,
b) determining energy dose in a timeslot with a reparation point in the centre of the timeslot, and proceeding with step c) for the timeslot if the energy dose in the timeslot is less than an energy dose limit, or proceeding with step g) if the energy dose in the timeslot is not less than the energy dose limit,
c) calculating a slope of the function in the timeslot to determine at least one measurement point, the at least one measurement point being positioned within the timeslot based on the slope of the function in the timeslot;
d) calculating a reparation dose at the measurement point, the reparation dose being the difference between the exposure energy dose applied at the at least one measurement point and a first energy dose threshold;
e) calculating a repair energy based on the reparation dose;
f) updating the function over the timeslot based on applying the repair energy at the reparation point; and
g) repeating steps b) to f) for the next reparation point until a loop through the function is completed.

2. The method of claim 1, further comprising setting the measurement point to be at least one of: earlier in time than a centre of the timeslot when the slope of the function is overall negative in the timeslot, later in time than a centre of the timeslot when the slope of the function is overall positive in the timeslot and/or in a centre of the timeslot when the function forms a valley in the timeslot.

3. The method of either of claims 1 or 2, wherein the slope of the function is determined in a first half of the timeslot and is determined in a second half of the timeslot.

4. The method of any preceding claim, further comprising setting a second energy dose threshold to be above 0% dose error or maximum energy dose of a peak of the function in the timeslot, and not updating the function if the energy dose in the timeslot would exceed the second energy dose threshold when the repair energy was applied at the reparation point.

5. The method of claim 4, further comprising incrementally increasing energy until the second energy dose threshold is reached, and keeping the previously accumulated energy.

6. The method of any preceding claim, further comprising setting the first energy dose threshold to be at least one of 0% dose error, in a range of -1 to 0% dose error, in a range of -5% to 0% dose error, in a range of -10% to 0% dose error.

7. The method of any of claims 1-5, further comprising setting the first energy dose threshold dependent on maximum energy dose in the timeslot.

8. The method of claim 7, further comprising reducing the first energy dose threshold if the maximum energy dose in the timeslot is greater than a predetermined energy dose.

9. The method of either of claims 7 or 8, further comprising setting the first energy dose threshold to be less than 0% dose error if the maximum energy dose in the timeslot is greater than 0% dose error.

10. The method of any preceding claim, wherein the loop is a first loop and the method further comprising: carrying out steps b) to g) in a second loop through the function opposite to the direction taken in the first loop, preferably, setting the first energy dose threshold of the second loop to be higher than the first energy dose threshold of the first loop, and, more preferably, setting a second energy dose threshold of the second loop to be higher than the second energy dose threshold of the first loop.

11. The method of any preceding claim, further comprising calculating the repair energy by incrementally increasing energy until the first energy dose threshold is reached.

12. The method of any preceding claim, further comprising applying, by a lithographic apparatus, the repair energy at one or more of the reparation points based on the reparation dose to repair the at least one die of the substrate.

13. A method for repairing at least one die comprising:
performing the method for calculating at least one reparation dose of any preceding claim, and
applying repair energy in at least one part of the die based on the reparation dose calculated.

14. A system configured for calculating at least one reparation dose for at least one die of a substrate exposed by a lithographic apparatus, the system configured to:
a) obtain a function of a distribution of an energy dose applied to the at least one die over time based on a measurement of exposure energy,
b) determine the energy dose in a timeslot with a reparation point in the centre of the timeslot, and proceed with step c) for the timeslot if the energy dose in the timeslot is less than an energy dose limit, or proceed with step g) if the energy dose in the timeslot is not less than the energy dose limit,
c) calculate a slope of the function in the timeslot to determine at least one measurement point, the at least one measurement point being positioned within the timeslot based on the slope of the function in the timeslot;
d) calculate a reparation dose at the measurement point, the reparation dose being the difference between the exposure energy dose applied at the at least one measurement point and a first energy dose threshold;
e) calculate a repair energy based on the reparation dose;
f) update the function over the timeslot based on applying the repair energy at the reparation point; and
g) repeat steps b) to f) for the next reparation point until a loop through the function is completed.

15. A lithographic apparatus comprising a projection system configured to project a EUV or DUV radiation beam to project a pattern from a patterning device onto a substrate, wherein the lithographic apparatus comprises the system of claim 14.
